# EUROPÄISCHE PATENTANMELDUNG

(11) **EP 2 388 026 A1**
(43) Veröffentlichungstag der Anmeldung: **23.11.2011**
(21) Anmeldenummer: 10005123.4
(22) Anmeldetag: 17.05.2010
(51) Int. Cl.: A61M 1/06, A61M 1/00, A61J 11/00

(54) **Vorrichtung zum Stillen eines Säuglings**

(71) Anmelder: Tritsch-Olian, Alexander, 53578 Windhagen (DE); Derfler, Frederic, 53639 Königswinter (DE)
(72) Erfinder: Tritsch-Olian, Alexander, 53578 Windhagen (DE); Derfler, Frederic, 53639 Königswinter (DE)
(74) Vertreter: Müller-Gerbes Wagner Albiger Patentanwälte

(57) **Zusammenfassung**

Die Erfindung betrifft eine Vorrichtung (1) zum Stillen eines Säuglings, umfassend ein Stillhütchen (3), das einen Rand (4) zur Anlage an eine Mutterbrust (2) aufweist, einen mit dem Stillhütchen verbundenen Sauger (8) für den Säugling, einen Milchspeicher (6), der bei angelegtem Stillhütchen (3) mit der Mutterbrust (2) verbunden ist, und eine Pumpe (16), die durch eine Unterdruckleitung (12) mit dem Milchspeicher (6) und/oder der Mutterbrust (2) verbunden ist und durch die ein Saugunterdruck (p_{S}) erzeugbar ist, um Milch aus der Mutterbrust (2) zu saugen, wobei der Milchspeicher (6) mit dem Sauger (8) über eine Saugleitung (10) mit dem Sauger (8) verbunden ist, wobei die Saugleitung (10) von Unterdruckleitung (12) und Pumpe (16) getrennt ist.

## Beschreibung

Die Erfindung betrifft eine Vorrichtung zum Stillen eines Säuglings, welche ein Stillhütchen mit einem Rand zur Anlage an einer Mutterbrust und einen mit dem Stillhütchen verbundenen Sauger für den Säugling aufweist.

Eine derartige Vorrichtung ist beispielsweise aus der DE 20 2004 003 825 U1 bekannt. Durch eine Pumpe, die durch eine Unterdruckleitung und durch das Stillhütchen mit der Mutterbrust verbunden ist, lässt sich ein Saugunterdruck erzeugen, um Milch aus der Mutterbrust zu saugen. Die Pumpe ist dabei zwischen Unterdruckleitung und dem Sauger angeordnet, wobei die Gefahr besteht, dass durch das Betätigen der Pumpe, die bevorzugt in der DE 20 2004 003 825 U1 als Quetschpumpe ausgebildet ist, Milch in den Mund des Säuglings gespritzt wird. Der Säugling kann somit nur schwerlich seinen natürlichen Saugrhythmus beibehalten, was das Stillen erschwert.

Die DE 395 101 zeigt ebenfalls eine Vorrichtung zum Stillen eines Säuglings, welche ein Stillhütchen, einen Sauger und eine Pumpe aufweist. Das auf die Mutterbrust anzulegende Stillhütchen begrenzt dabei einen Milchspeicher, der mit Milch gefüllt wird, wenn die Pumpe betätigt wird. Aber auch hier besteht das Problem, dass bei Betätigung der Pumpe Milch über den Sauger in den Mund des Säuglings gespritzt wird. Dies steht jedoch, wie oben bereits ausgeführt, einem natürlichen und erfolgreichen Stillen des Säuglings entgegen.

Der Erfindung liegt daher die Aufgabe zugrunde, eine Vorrichtung zum Stillen eines Säuglings bereitzustellen, die ein Stillen des Säuglings möglichst ohne negative Nebenwirkungen erleichtert.

Die der Erfindung zugrunde liegende Aufgabe wird mit der Merkmalskombination gemäß Patentanspruch 1 gelöst. Bevorzugte Ausführungsbeispiele können den Unteransprüchen entnommen werden.

Die Vorrichtung gemäß Patentanspruch 1 zeichnet sich dadurch aus, der Milchspeicher mit dem Sauger über eine Saugleitung mit dem Sauger verbunden ist, wobei die Saugleitung von Unterdruckleitung und Pumpe getrennt ist. Dadurch ist es nicht möglich, dass die Pumpe einen Druck aufbaut, durch den Milch in den Mund des Säuglings gespritzt wird. Vorzugsweise sind ein Anschluss der Unterdruckleitung und ein Anschluss oder ein speicherseitiges Ende der Saugleitung an entgegen gesetzten Enden des Milchspeichers angeordnet. In Einsatzlage der Vorrichtung ist bevorzugt vorgesehen, dass in die Unterdruckleitung keine Milch gelangen kann. Zudem ist die Pumpe so anzuordnen und einzusetzen, dass sie lediglich einen Unterdruck im Milchspeicher oder in der Unterdruckleitung erzeugen kann, jedoch nicht einen Überdruck in der Saugleitung.

Vorzugsweise ist eine manuell betätigbare oder durch eine Steuereinheit ansteuerbare Entlüftung vorgesehen ist, um Unterdruck im Milchspeicher abzubauen. Auf diese Weise ist es möglich, dass der Säugling Milch aus dem Milchspeicher entnimmt, ohne gegen den im Milchspeicher herrschenden Unterdruck anzukämpfen. Der Säugling kann dabei in seinem natürlichen Saugrhythmus trinken, wobei seine Saugleistung gegenüber der Leistung eines natürlichen Stillens ohne die erfindungsgemäße Vorrichtung deutlich reduziert werden kann.

Die Entlüftung kann ein Entlüftungsventil umfassen, welches in der Unterdruckleitung oder am Milchspeicher vorgesehen ist. Ist das Entlüftungsventil geöffnet, kann der Säugling ohne große Anstrengung Milch über den Sauger aus dem Milchspeicher entnehmen. Bei geschlossenem Entlüftungsventil und nicht laufender Pumpe muss der Säugling in dem Maße saugen, wie es bei einem Stillen ohne die erfindungsgemäße Vorrichtung notwendig wäre. Die stillende Mutter kann somit bei einer manuell betätigbaren Entlüftung entscheiden, mit welchem Kraftaufwand der Säugling Milch zu sich nehmen soll.

In einem bevorzugten Ausführungsbeispiel sind Mittel zum Erfassen mindestens eines Parameters vorgesehen, aus dem ableitbar ist, ob der Säugling Milch zu sich nehmen möchte oder bereits zu sich nimmt. Beispielsweise kann dies ein Trink-Unterdruck sein, der sich in der Saugleitung oder im Sauger einstellt. Alternativ oder zusätzlich könnte der Parameter eine Durchflussmenge sein, die in der Saugleitung oder im Sauger gemessen wird. Wird beispielsweise durch den Trink-Unterdruck erfasst, dass der Säugling stark saugt und Milch zu sich nehmen möchte, kann die Entlüftung geöffnet werden, so dass der Säugling in einfacher Weise Milch aus dem Milchspeicher entnehmen kann. Soweit in diesem Fall die Pumpe läuft, kann in Abhängigkeit des Trink-Unterdrucks die Pumpe zusätzlich abgestellt werden.

Vorzugsweise stehen die Mittel zum Erfassen des Trink-Parameters mit der Steuereinheit in Verbindung, die ausgelegt ist, in Abhängigkeit des erfassten Trink-Parameters die Pumpe und/oder die Entlüftung anzusteuern. Somit ist es möglich, dass automatisch die Pumpe gestoppt und die Entlüftung geöffnet wird, wenn der Säugling beginnt zu trinken.

Vorzugsweise ist der Milchspeicher bei angelegtem Stillhütchen durch die Mutterbrust und eine Innenwandung des Stillhütchens begrenzt. Dies hat den Vorteil, dass die im Milchspeicher befindliche Milch durch die Mutterbrust auf Körpertemperatur gehalten wird. Der Säugling kann somit Muttermilch mit einer Temperatur wie beim Stillen ohne die erfindungsgemäße Vorrichtung zu sich nehmen. Natürlich ist es auch möglich, Stillhütchen und Milchspeicher räumlich voneinander zu trennen. In diesem Fall würde aber die Milch nicht durch die Mutterbrust gewärmt werden.

In einem bevorzugten Ausführungsbeispiel ist ein Timer vorgesehen, wodurch die Pumpe und/oder die Entlüftung in Abhängigkeit des Trink-Parameters um eine Zeitspanne Δt zeitlich verzögert reagiert. Legt beispielsweise der Säugling beim Trinken eine Trinkpause ein, so läuft die zuvor abgeschaltete Pumpe erst nach einer gewissen Zeitspanne an. Somit ist es dem Säugling möglich, eine Trinkpause einzulegen, ohne dass dadurch die Pumpe anläuft und die Druckverhältnisse im Milchspeicher geändert werden. Vorzugsweise beträgt die Zeitspanne 2 bis 4 sec. Der Timer kann dabei separat ausgebildet sein oder Teil der Steuereinheit sein. Vorzugsweise ist die Zeitspanne von außen einstellbar, so dass die erfindungsgemäße Vorrichtung auf die individuellen Trinkgewohnheiten eines Säuglings eingestellt werden kann.

Es sei an dieser Stelle darauf hingewiesen, dass unabhängig von den Mitteln zum Erfassen mindestens eines Trink-Parameters, ob der Säugling Milch zu sich nehmen möchte oder zu sich nimmt, ein Sensor zur Erfassung der vom Säugling aufgenommenen Milchmenge vorgesehen ist. Bevorzugt ist dabei dieser Sensor in der Saugleitung untergebracht. Bei Erreichen der Obergrenze für die Durchflussmenge kann ein Signal akustischer, visueller und/oder haptischer Natur erzeugt werden, so dass die stillende Mutter entscheiden kann, ob der Säugling weiter gestillt werden soll oder nicht.

Vorzugsweise sind Mittel zum Erfassen einer Milchmenge im Milchspeicher, beispielsweise in Form eines Schwimmers, vorgesehen. Somit soll sichergestellt werden, dass die Pumpe gestoppt wird, wenn der Milchspeicher voll gelaufen ist oder einen bestimmten Füllgrad aufweist. Zudem kann damit auch der Zustand eines leeren Milchspeichers detektiert werden, damit die Pumpe aktiviert werden kann. Vorzugsweise sind die Mittel zum Erfassen der Milchmenge mit der Steuereinheit verbunden, so dass die Pumpe in Abhängigkeit der Milchmenge im Milchspeicher automatisch anläuft oder gestoppt wird.

Der Milchspeicher kann einen Anschluss für einen externen Milchbehälter aufweisen. Somit ist es möglich, Milch aus dem Milchbehälter über die Pumpe in den Milchspeicher zu saugen, die dann über die Saugleitung und den Sauger zum Säugling gelangt. Auch hier kann wieder über die Steuereinheit sichergestellt werden, dass der Milchspeicher immer gefüllt ist und ein Trinken des Säuglings aus dem gefüllten Milchspeicher möglich ist. Bei einem Überangebot von Milch lässt sich der externe Milchbehälter zum Aufbewahren der Milch füllen.

In einem bevorzugten Ausführungsbeispiel ist zwischen dem Sauger und der Saugleitung ein Zwischenventil vorgesehen. Das Zwischenventil verhindert dabei, dass Luft aus dem Mund des Säuglings gezogen wird, wenn die Pumpe arbeitet. Ist beispielsweise der Milchspeicher entleert und die Pumpe läuft an, um diesen wieder zu füllen, ist das Zwischenventil geschlossen. Der Säugling kann dann keine Milch aus dem Milchspeicher entnehmen. Das Zwischenventil kann dabei so ausgelegt sein, dass es schließt, wenn der durch die Pumpe erzeugte Unterdruck größer ist als der durch den Säugling erzeugte Unterdruck am Sauger.

Die Pumpe kann ein Eintrittsventil und ein Austrittsventil aufweisen. Dadurch lässt sich auch die Pumpe entlüften.

Das erfindungsgemäße Verfahren bietet somit die folgenden Vorteile:
a) der Säugling trinkt natürlich von der Mutterbrust;
b) die Muttermilch fließt nicht durch die Pumpe;
c) die Pumpe bleibt sauber, eine ohnehin sehr schwierige Sterilisation der Pumpe ist daher nicht notwendig;
d) die Vorrichtung bleibt sauber und hygienisch;
e) die Milchqualität ist hoch, bleibt sauber und warm; und
f) die Vorrichtung ermöglicht es dem Säugling permanent zu saugen, unabhängig von der Pumpe und der Säugling kann seinen natürlichen Saugrhythmus individuell beibehalten.

Nach Bedarf des Säuglings kann, wie oben bereits ausgeführt, die Pumpe automatisch geregelt bzw. angesteuert werden:
a) wenn der Säugling trinkt, wird die Pumpe automatisch ausgeschaltet;
b) wenn der Säugling eine Trinkpause einlegt, schaltet sich die Pumpe erst nach einer bestimmten Zeit automatisch wieder ein;
c) durch die Pumpe wird der Milchspeicher wieder aufgefüllt, so dass Muttermilch leicht zum Säugling gelangen kann, um diesen entsprechend zu stimulieren, das Trinken aufzunehmen oder beizubehalten.

Durch Verwendung des Milchspeichers steht dem Säugling eine größere Milchmenge zum Trinken zur Verfügung, die er ohne große Kraftanstrengung zu sich nehmen kann.

Soweit der Milchspeicher direkt an der Mutterbrust angebracht ist, wird die im Milchspeicher befindliche Milch auf natürlichem Wege warm gehalten. Bei geöffnetem Entlüftungsventil kann die Muttermilch aus dem Milchspeicher in einfacher Weise zum Säugling gelangen, wodurch eine Stimulation und Anregung zum Trinken bzw. Saugen des Säuglings erzielt wird.

Die erfindungsgemäße Vorrichtung kann in unterschiedlicher Weise an der Mutterbrust angebracht werden. Beispielsweise kann ein Doppelklebestreifen verwendet werden, der zwischen Stillhütchen und Mutterbrust angeordnet wird. Eine andere Möglichkeit der Anbringung besteht darin, dass sich das Stillhütchen aufgrund von Unterdruck an der Mutterbrust festsaugt. Dafür ist es nötig, dass der Rand des Stillhütchens, der zur Anlage an die Mutterbrust dient, eine luftdichte Verbindung zwischen Mutterbrust und Stillhütchen ermöglicht. Zudem kann die erfindungsgemäße Vorrichtung durch einen speziellen Büstenhalter an der Mutterbrust angebracht werden.

Anhand der in den Figuren dargestellten Ausführungsbeispiele wird die Erfindung näher erläutert. Es zeigen:
- Figur 1: ein erstes Ausführungsbeispiel an einer Mutterbrust;
- Figur 2: eine Vergrößerung des Ausschnitts II der Figur 1; und
- Figur 3: schematisch diverse Bauteile eines zweiten Ausführungsbeispiels der Erfindung.

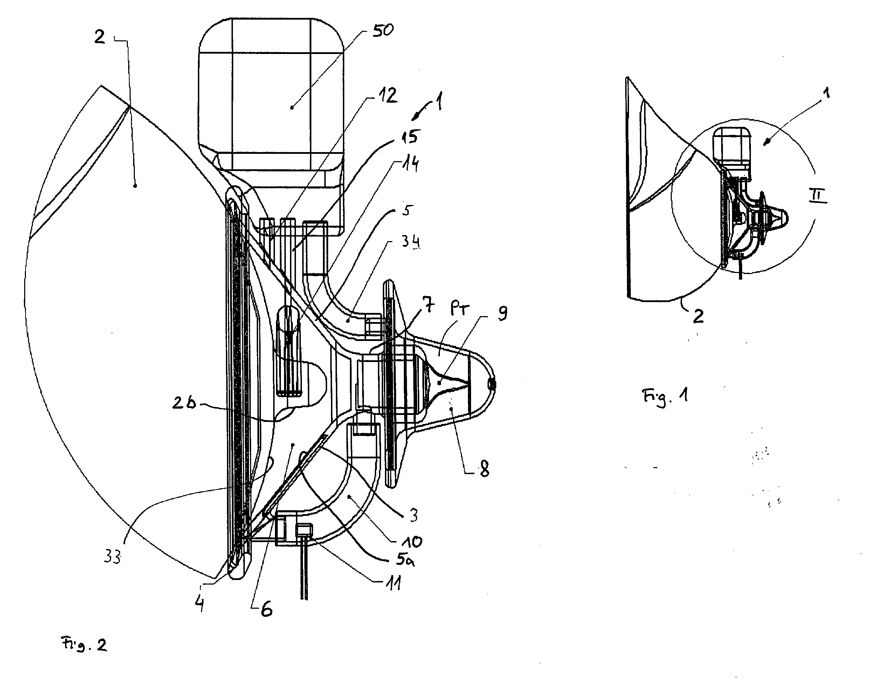

Figur 1 zeigt eine Vorrichtung zum Stillen eines Säuglings, welche in ihrer Gesamtheit mit 1 bezeichnet wird. Die Vorrichtung 1 liegt an einer Mutterbrust 2 an. Figur 2, auf die im Folgenden Bezug genommen wird, zeigt eine Vergrößerung des kreisförmigen Ausschnitts II der Figur 1.

Die Vorrichtung 1 umfasst ein Stillhütchen 3 mit einem Rand 4, der im Wesentlichen ringförmig ist und an der Mutterbrust 2 anliegt. Der Ring 4 umschließt eine runde gewölbte, in der Mitte ein Loch aufweisende Membran 33, die flächig auf der Mutterbrust 2 liegt. Der Rand 4 kann beispielsweise aus Kunststoff sein, welches die Form der Mutterbrust 2 gut annimmt. Das Stillhütchen 3 weist eine im Wesentlichen kegelförmige Form auf, wobei eine Mantelfläche 5 des Stillhütchens 3 ebenfalls elastisch ausgebildet ist und von einem starren Skelett (nicht dargestellt) gehalten wird. Alternativ kann die Mantelfläche 5 starr ausgebildet sein, wobei dann auf das Skelett verzichtet werden kann.

Eine Innenwandung 5a des Stillhütchens 3 bzw. der kegelförmigen Mantelfläche 5 und die Membran 33, der von dem ringförmigen Rand 4 umschlossen ist, begrenzen einen Milchspeicher 6. Dadurch wird der Milchspeicher 6 geformt.

An einer Spitze 7 des Stillhütchens 3 ist ein vorzugsweise aus Silikon hergestellter Sauger 8 für den zu stillenden Säugling angebracht. Der Sauger 8 ist vorzugsweise separat gefertigt ist und wird auf die Spitze 7 des Stillhütchens gesteckt oder sonst wie lösbar befestigt, wodurch der Sauger 8 abgenommen und einfach sterilisiert werden kann. In dem Sauger 8 ist ein Zwischenventil 9 angeordnet, das den am Sauger 8 saugenden Säugling von dem Milchspeicher 6 trennt, soweit ein Unterdruck im Milchspeicher größer ist als ein Unterdruck im Sauger 8. Das Zwischenventil sorgt dafür, dass der Säugling nicht erstickt, wenn die Pumpe läuft.

Milchspeicher 6 und Sauger 8 sind durch eine Saugleitung 10 miteinander verbunden. In der Saugleitung 10 ist ein Sensor 11 für eine Mengenmessung durch die Saugleitung 10 angeordnet. Durch den Sensor 11 für die Mengenmessung erhält man somit die Information, wie viel Milch der Säugling zu sich genommen hat.

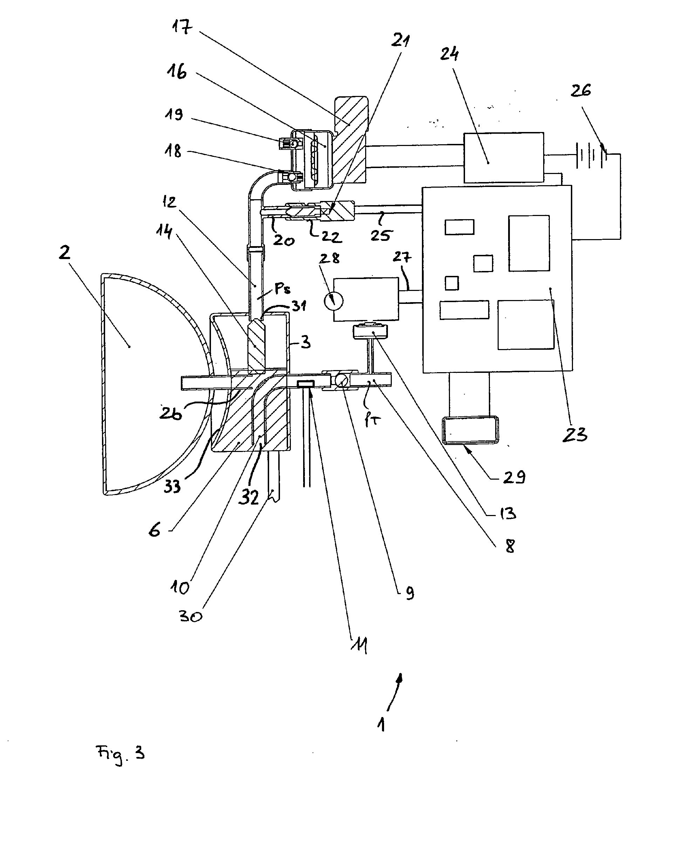

Eine Einheit 50, die aus mehreren Komponenten der Vorrichtung 1 besteht, auf die in Einzelnen bei der Figurenbeschreibung der Figur 3 eingegangen wird, ist mit dem Milchspeicher 6 über eine Unterdruckleitung 12 verbunden. Die Einheit 50 ist über eine Vakuumleitung 34 und einen Vakuumsensor 13 (in Figur 3 dargestellt) mit dem Sauger 8 verbunden. Mit dem Vakuumsensor 13 lässt sich ein Trink-Unterdruck p_{T} erfassen, der sich im Sauger einstellt, wenn der Säugling an dem Sauger 8 saugt und Milch zu sich nehmen möchte.

Des Weiteren steht die Einheit 50 mit Mittel zum Erfassen einer Milchmenge im Milchspeicher 6 in Form eines Schwimmers 14 in Wirkverbindung, der innerhalb des Milchspeichers 6 angeordnet ist. Über einen zwischen Schwimmer 14 und Einheit 50 geschalteten Anschluss 15 erfolgt eine Kopplung zwischen dem Schwimmer 14 und der Einheit 50. Der Schwimmer ist im Ausführungsbeispiel der Figur 2 C-förmig ausgebildet und umgreift in einem Winkelbereich von ca. 180° eine Brustwarze 2b der Mutterbrust 2.

Wie oben bereits ausgeführt, umfasst die Einheit 50 mehrere Komponenten, auf die nun näher unter Bezugnahme auf Figur 3 näher eingegangen wird.

Figur 3 zeigt in schematisierter Weise die erfindungsgemäße Vorrichtung 1. Die oben bereits erwähnte Einheit 50 umfasst insbesondere eine Pumpe 16, die durch einen Motor 17 angetrieben wird. Die Pumpe 16 weist ein Eintrittsventil 18 und ein Austrittsventil 19 auf, wobei Letztgenanntes mit einem Schalldämpfer ausgestattet sein kann.

Merkmale oder Bauteile des Ausführungsbeispiels der Figur 3, die den Bauteilen und Merkmalen des Ausführungsbeispiels der Figur 2 ähnlich sind oder entsprechen, werden mit entsprechend gleichen Bezugszeichen belegt. So ist beispielsweise die Pumpe 16, die im Ausführungsbeispiel der Figur 2 Teil der Einheit 50 ist, über die Unterdruckleitung 12 mit dem Milchspeicher 6 verbunden. Die Unterdruckleitung 12 weist dabei einen Anschluss 20 für eine Entlüftung 21 auf, die ein Entlüftungsventil 22 umfasst. Ein geöffnetes Entlüftungsventil 22 stellt eine Verbindung zwischen der Unterdruckleitung und der Umgebung dar, wodurch ein Saugunterdruck p_{S} in der Unterdruckleitung und somit auch im Milchspeicher zusammenfällt.

Die Vorrichtung 1 umfasst des Weiteren eine Steuereinheit 23, die sowohl über einen Motorregler 24 mit dem Motor 17 und der daran befestigten Pumpe 16 als auch über eine Leitung 25 mit der Entlüftung 21 verbunden ist. An der Steuereinheit 23 ist des Weiteren eine Energiequelle 26 angeschlossen, die beispielsweise eine Batterie sein kann. Die Energiequelle 26 kann aber auch räumlich getrennt von der Vorrichtung 1 in Form einer Steckdose oder eines Transformators angeordnet sein, wobei dann für die Energiequelle 26 stellvertretend ein entsprechender Anschluss für eine externe Energiequelle gelten soll.

Die Steuereinheit 23 umfasst des Weiteren einen Anschluss 27 für einen Timer 28, der wiederum mit dem zuvor beschriebenen Vakuumsensor 13 verbunden ist. Letztlich sei noch im Zusammenhang mit der Steuereinheit 23 auf eine Anzeige 29 verwiesen, die die vom Säugling durch den Sauger 8 aufgenommene Milchmenge anzeigen soll. Die Milchmenge wird dabei durch den Sensor 11 für die Mengenmessung erfasst, wobei der Sensor 11 in der Saugleitung 10 angeordnet ist, die Milchspeicher 6 und Sauger 8 miteinander verbindet. Dem Sauger 8 vorgeschaltet ist das Zwischenventil 9, das als einfaches Kugelrückschlagventil ausgebildet sein kann. Das Zwischenventil 9 soll sicherstellen, dass in dem Milchspeicher 6 Unterdruck aufgebaut werden kann, wenn die Pumpe durch die Unterdruckleitung 12 Luft ansaugt.

Die Vorrichtung 1 gemäß Figur 3 weist des Weiteren einen Anschluss 30 für einen externen Milchbehälter auf. Je nach Art und Weise, wie die Vorrichtung 1 betrieben werden soll, kann der Milchbehälter 30 leer oder mit Milch gefüllt sein.

Wie im Ausführungsbeispiel der Figur 2 befindet sich der Schwimmer 14 innerhalb des Milchspeichers 6, der, dargestellt durch die Schrägschraffur, ungefähr halb mit Milch gefüllt ist. Der Schwimmer 14 nimmt bei einem derartigen Füllstand eine Stellung ein, durch die er eine Öffnung 31 der Unterdruckleitung 12 zum Milchspeicher 6 schließt. Dadurch sind Unterdruckleitung 12 und Milchspeicher 6 voneinander getrennt, was zur Folge haben kann, dass bei laufender Pumpe 16 der Unterdruck p_{S} in der Unterdruckleitung 12 schnell ansteigt und zu einer automatischen Abschaltung der Pumpe führt.

Letztlich wird auf die stark schematisierte Mutterbrust 2 mit der Brustwarze 2b verwiesen, wobei die Darstellung der Brustwarze 2b nur auf deren Funktion beschränkt ist, eine Verbindung zwischen Mutterbrust 2 und dem Milchspeicher 6 herzustellen. Das Stillhütchen 3 ist in Figur 3 ebenfalls stark schematisiert als im Wesentlichen zylindrischer Behälter dargestellt.

Im Folgenden soll nun beschrieben werden, wie die erfindungsgemäße Vorrichtung 1 verwendet werden kann. Zum Stillen des Säuglings wird das Stillhütchen 3 mit dem leeren Milchspeicher 6 an der Mutterbrust 2 zum Beispiel, wie oben bereits ausgeführt, mittels Doppelklebestreifen, Unterdruck anlegen und/oder Büstenhalter angebracht. Das Anlegen des Unterdrucks kann beispielsweise dadurch erfolgen, wenn auf die Ausführung des Stillhütchens 3 in der Figur 2 Bezug genommen wird, indem die Pumpe 16 aktiviert wird, wodurch bei angelegtem Stillhütchen 3 durch den Unterdruck in der Unterdruckleitung 12 und dann auch im Milchspeicher 6 das Stillhütchen 3 an die Mutterbrust 2 festgesaugt wird. Bei entsprechend großem Saugdruck pₛ tritt Muttermilch aus der Brustwarze 2b, wodurch der Milchspeicher 6 gefüllt wird.

Die Pumpe 16 erzeugt bevorzugt einen Unterdruckverlauf, die dem natürlichen Saugen des Säuglings in Zyklen entspricht. Auch der maximale Unterdruck ist dem Unterdruck angepasst oder ähnelt dem maximalen Unterdruck, der von einem durchschnittlichen Säugling erzeugt werden kann.

Die Pumpe 16 läuft dabei so lange, bis der Schwimmer 14 die Verbindung zwischen Unterdruckleitung 12 und Milchspeicher 6 schließt, wodurch es zu einem steilen Anstieg des Saugunterdrucks pₛ in der Saugleitung 12 kommt, was zu einer automatischen Abschaltung der Pumpe 16 führt. Der Säugling kann nun über den Sauger 8 an die Vorrichtung 1 mit dem gefüllten Milchspeicher 6 angelegt werden. Sobald der Vakuumsensor 13 einen Trink-Unterdruck p_{T} erfasst, wird über die Steuereinheit 23 die Entlüftung 21 geöffnet. Dadurch kann Luft durch das Entlüftungsventil 22 in die Unterdruckleitung 12 und dann in den Milchspeicher 6 gelangen, wodurch Unterdruckleitung 12 und Milchspeicher 6 auf Atmosphärendruck gelegt werden. Der Säugling kann nun mit vergleichsweise wenig Anstrengung die Milch aus dem Milchspeicher 6 entnehmen, da er nicht gegen irgendwelche Unterdrücke ankämpfen muss. Die Milch wird dabei durch die Saugleitung 10 durch das Zwischenventil 9 in den Sauger 8 befördert. Wie in Figur 2 und in Figur 3 zu erkennen ist, ist ein speicherseitiges Ende 32 der Saugleitung 10 in einem unteren Bereich des Milchspeichers 6 angeordnet, in dem sich nur bei vollständig entleertem Milchspeicher 6 keine Milch befindet. Das speicherseitige Ende 31 der Unerdruckleitung 12 ist dabei im oberen Bereich des Milchspeichers angeordnet. Die Pumpe 16 kann somit Milch nicht aus dem Mund des Säuglings saugen, zumal das Zwischenventil 9 vorgesehen ist. Auch ist ein Einspritzen von Milch in den Mund des Säuglings ausgeschlossen.

Wie in Figur 2 zu erkennen, kann der Schwimmer 14 eine Position einnehmen, in der er die Öffnung 31 der Unterdruckleitung 12 schließt. Dies ist als eine zusätzliche Maßnahme gedacht, die Milchmenge in dem Milchspeicher zu begrenzen, falls die Steuereinheit 23 aufgrund eines Fehlers die Pumpe 16 nicht stoppt.

Die Milchmenge, die durch die Saugleitung 10 fließt, wird durch den Sensor 11 erfasst. Der Sensor 11 sendet dabei Signale (beispielsweise über eine hier nicht dargestellte Leitung) zur Steuereinheit 23, die diese umsetzt und über die Anzeige 29 als ablesbare Milchmenge visualisiert.

Soweit aus den Signalen des Sensors 11 für die Mengenmessung und/oder aus den Signalen des Vakuumsensors 13 für den Trink-Unterdruck p_{T} hervorgeht, dass der Säugling Milch zu sich nimmt, bleibt die Pumpe deaktiviert. Dies kann auch den Zustand umfassen, bei dem der Säugling so stark saugt, dass der im Sauger 8 erzeugte Trink-Unterdruck p_{T} so groß ist, dass er über die Saugleitung 10 und den Milchspeicher 6 Milch aus der Mutterbrust 2 zu saugen vermag. Damit wäre ein wichtiges Ziel der Vorrichtung 1 erreicht: Durch die durch die Pumpe 16 in den Milchspeicher 6 gesaugte Milchmenge wird es dem Säugling leicht gemacht, die ersten Tropfen Milch zu sich zu nehmen. Dadurch wird der Säugling stimuliert und angeregt und kann dadurch einen genügend großen Unterdruck aufbauen, um selbstständig Muttermilch aus der Mutterbrust 2 zu ziehen.

Nimmt jedoch der Säugling keine Milch mehr zu sich, nuckelt nur noch an dem Sauger oder droht beim Stillen einzuschlafen, fällt der Trink-Unterdruck p_{T} im Sauger 8 zusammen, was von dem Vakuumsensor 13 erfasst wird. Handelt es sich nicht nur um eine kurze Trinkpause des Säuglings, die beispielsweise zwei oder drei Sekunden betragen kann, aktiviert die Steuereinheit 23 über den Motorregler 24 und den Motor 17 die Pumpe 16. Gleichzeitig wird das Entlüftungsventil 22 geschlossen, so dass sich dann in der Unterdruckleitung 12 und damit auch im Milchspeicher 6 ein Saug-Unterdruck p_{S} einstellt. Durch diesen Unterdruck wird nun wiederum Milch aus der Mutterbrust 2 gesaugt, so dass sich der Milchspeicher 6 füllt. Ein gefüllter Milchspeicher 6 wiederum aber erleichtert es dem Säugling wesentlich, Milch zu sich zu nehmen, so dass ein trinkfauler Säugling oder ein Säugling, der in den Schlaf zu fallen droht, weiter Milch zu sich nehmen kann und der Stillvorgang nicht vorzeitig beendet wird.

Der Stillvorgang kann von beendet werden, wenn die erfasste Milchmenge, die durch die Saugleitung 10 in den Sauger 8 und damit zum Säugling geführt worden ist, eine bestimmte Obergrenze erreicht hat. Vorzugsweise entscheidet allein die Mutter über die Beendigung des Stillvorgangs, sodass die Obergrenze nur als informative Anzeige zu verstehen ist. Etwaige überschüssige Milch in der Mutterbrust 2 kann nach dem Stillen des Säuglings über den Anschluss 30 in den externen Milchbehälter geführt werden.

Zu einem späteren Zeitpunkt kann die somit in den externen Milchbehälter gelangte Milch wieder zum Stillen genutzt werden. Dafür wird der gefüllte Milchbehälter über den Anschluss 30 an den Milchspeicher 6 angeschlossen. Die Pumpe saugt nun Milch, sei es ausschließlich aus dem externen Milchbehälter, sei es zusätzlich aus der Mutterbrust, und füllt somit den Milchspeicher 6. Die in den Milchspeicher 6 gelangte Milch kann, wie oben bereits beschrieben, in einfacher Weise von dem Säugling aufgenommen werden.

Es versteht sich, dass über den Anschluss 30 auch ein Muttermilchersatz, Milch einer anderen Mutter oder einer anderen Frau, oder auch Additive dem Milchspeicher 6 zugeführt werden können, die der Säugling zu sich nehmen soll.

Die erfindungsgemäße Vorrichtung erleichtert somit ein Stillen aller Säuglinge, insbesondere trinkfauler oder geschwächter Säuglinge. Zudem kann die erfindungsgemäße Vorrichtung auch zum Schutz der Brustwarze 2b oder der Mutterbrust 2 eingesetzt werden, wenn die stillende Mutter eine platte oder negative Brustwarze hat oder die Haut der Mutterbrust trocken ist oder Risse/Entzündungen aufweist. Auch kann die erfindungsgemäße Vorrichtung als ausgleichendes Mittel für Mütter eingesetzt werden, die zu wenig oder zu viel Milch haben.

Somit vereint die erfindungsgemäße Vorrichtung eine Vielzahl von Vorteilen, die das Stillen eines Säuglings entscheidend vereinfachen. Durch das gezielte Anstillen des Säuglings und der unterstützenden Wirkung der Pumpe 16 beim Stillen ist der Stillvorgang besser planbar. Die Stillzeit lässt sich dramatisch kürzen, was insbesondere viel beschäftigte Mütter zu Gute kommt. Durch die Milchmengenmessung lässt sich der Stillvorgang in einfacher Weise von der Mutter überwachen. Insgesamt führt die erfindungsgemäße Vorrichtung zu einem Stillen ohne Stress, was wiederum das Stillen für Mutter und Säugling weiter erleichtert.

### Bezugszeichenliste

- 1: Vorrichtung
- 2: Mutterbrust
- 3: Stillhütchen
- 4: Rand
- 5: Mantelfläche
- 5a: Innenfläche der Mantelfläche
- 6: Milchspeicher
- 7: Spitze
- 8: Sauger
- 9: Zwischenventil
- 10: Saugleitung
- 11: Sensor für Mengenmessung
- 12: Unterdruckleitung
- 13: Vakuumsensor
- 14: Schwimmer
- 15: Anschluss
- 16: Pumpe
- 17: Motor
- 18: Eintrittsventil
- 19: Austrittsventil
- 20: Anschluss
- 21: Entlüftung
- 22: Entlüftungsventil
- 23: Steuereinheit
- 24: Motorregler
- 25: Leitung
- 26: Energiequelle
- 27: Leitung
- 28: Timer
- 29: Anzeige
- 30: Anschluss
- 31: Öffnung
- 32: Öffnung
- 33: Membran
- 34: Vakuumleitung
- 50: Einheit

## Patentansprüche

1. Vorrichtung (1) zum Stillen eines Säuglings, umfassend
- ein Stillhütchen (3), das einen Rand (4) zur Anlage an eine Mutterbrust (2) aufweist;
- einen mit dem Stillhütchen verbundenen Sauger (8) für den Säugling;
- einen Milchspeicher (6), der bei angelegtem Stillhütchen (3) mit der Mutterbrust (2) verbunden ist; und
- eine Pumpe (16), die durch eine Unterdruckleitung (12) mit dem Milchspeicher (6) und/oder der Mutterbrust (2) verbunden ist und durch die ein Saug-unterdruck (p_{S}) erzeugbar ist, um Milch aus der Mutterbrust (2) zu saugen,
**dadurch gekennzeichnet, dass** der Milchspeicher (6) mit dem Sauger (8) über eine Saugleitung (10) mit dem Sauger (8) verbunden ist, wobei die Saugleitung (10) von Unterdruckleitung (12) und Pumpe (16) getrennt ist.

2. Vorrichtung (1) nach Anspruch 1, **dadurch gekennzeichnet, dass** eine manuell betätigbare oder durch eine Steuereinheit (23) ansteuerbare Entlüftung (21) vorgesehen ist, um Unterdruck im Milchspeicher (6) abzubauen.

3. Vorrichtung (1) nach Anspruch 2, **dadurch gekennzeichnet, dass** die Entlüftung (21) ein Entlüftungsventil (22) umfasst, welches an der Unterdruckleitung (12) oder am Milchspeicher (6) vorgesehen ist.

4. Vorrichtung (1) nach einem der Ansprüche 1 bis 3, **dadurch gekennzeichnet, dass** Mittel zum Erfassen mindestens eines Trink-Parameters vorgesehen sind, aus dem ableitbar ist, ob der Säugling Milch zu sich nimmt oder zu sich nehmen möchte.

5. Vorrichtung (1) nach Anspruch 4, **dadurch gekennzeichnet, dass** die die Mittel zum Erfassen des Trink-Parameters einen Vakuum- oder Unterdrucksensor (13) umfassen, der einen Trink-Unterdruck (p_{T}) erfasst, der sich in der Saugleitung (10) oder im Sauger einstellt.

6. Vorrichtung (1) nach Anspruch 4 oder 5, **dadurch gekennzeichnet, dass** die Mittel zum Erfassen des Trink-Parameters mit der Steuereinheit in Verbindung stehen, die ausgelegt ist, in Abhängigkeit des erfassten Trink-Parameters die Pumpe und/oder das Entlüftungsventil anzusteuern.

7. Vorrichtung (1) nach einem der Ansprüche 1 bis 6, **dadurch gekennzeichnet, dass** der Milchspeicher (6) bei angelegtem Stillhütchen (3) durch die Mutterbrust (2) und eine Innenwandung (5a) das Stillhütchen (3) begrenzt ist.

8. Vorrichtung (1) nach einem der Ansprüche 4 bis 7, **dadurch gekennzeichnet, dass** ein Timer (28) vorgesehen ist, wodurch die Pumpe (16) und/oder die Entlüftung (21) in Abhängigkeit des Trink-Parameters um eine Zeitspanne Δt zeitlich verzögert reagiert.

9. Vorrichtung (1) nach einem der Ansprüche 1 bis 8, **dadurch gekennzeichnet, dass** Mittel (14) zum Erfassen einer Milchmenge im Milchspeicher (6) vorgesehen sind.

10. Vorrichtung (1) nach Anspruch 9, **dadurch gekennzeichnet, dass** die Mittel (14) zum Erfassen der Milchmenge mit der Steuereinheit (23) verbunden sind.

11. Vorrichtung (1) nach einem der Ansprüche 1 bis 10, **dadurch gekennzeichnet, dass** der Milchspeicher (6) einen Anschluss (30) für einen externen Milchbehälter aufweist.

12. Vorrichtung (1) nach einem der Ansprüche 1 bis 11, **dadurch gekennzeichnet, dass** zwischen Sauger (8) und Saugleitung (10) ein Zwischenventil vorgesehen ist.

13. Vorrichtung (1) nach einem der Ansprüche 1 bis 12, **dadurch gekennzeichnet, dass** die Pumpe (16) ein Eintrittsventil (18) und ein Austrittsventil (19) aufweist.
